(19) Europäisches Patentamt
European Patent Office
Office européen des brevets

(11) Numéro de publication: **0 130 895**
**B1**

# (12) FASCICULE DE BREVET EUROPÉEN

(45) Date de publication du fascicule du brevet: **05.11.86**

(51) Int. Cl.⁴: **C 07 C 29/15,** C 07 C 31/04, C 25 B 3/04

(21) Numéro de dépôt: **84401316.9**

(22) Date de dépôt: **22.06.84**

(54) **Procédé de préparation du méthanol par hydrogénation du monoxyde de carbone en présence d'un catalyseur constitué par un polymère organique conducteur tel que le polypyrrole.**

(30) Priorité: **29.06.83 FR 8310774**

(43) Date de publication de la demande: **09.01.85 Bulletin 85/2**

(45) Mention de la délivrance du brevet: **05.11.86 Bulletin 86/45**

(84) Etats contractants désignés: **BE DE GB IT NL SE**

(56) Documents cité: **FR-A-2 314 209** **GB-A-1 487 665**

(73) Titulaire: **COMMISSARIAT A L'ENERGIE ATOMIQUE Etablissement de Caractère Scientifique Technique et Industriel, 31/33, rue de la Fédération, F-75015 Paris (FR)**

(72) Inventeur: **Genies, Eugène, La Biolette Rue Troussai, F-38120 Saint Egreve (FR)**
Inventeur: **Szamos, Agnès, 1221 Rozsa PU. 20- 22, Budapest (HU)**

(74) Mandataire: **Mongrédien, André, c/o BREVATOME 25, rue de Ponthieu, F-75008 Paris (FR)**

**EP 0 130 895 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

**Description**

La présente invention a pour objet un procédé de préparation du méthanol par hydrogénation catalytique du monoxyde de carbone.

De façon plus précise, elle concerne de nouveaux catalyseurs d'hydrogénation susceptibles d'être utilisés dans un tel procédé.

On sait que les besoins en méthanol de l'industrie chimique sont très importants. En effet, le méthanol est utilisé comme solvant dans de nombreuses réactions industrielles et comme produit de départ pour la synthèse de divers composés, en particulier du formaldéhyde. Par ailleurs, il est utilisé comme additif pour abaisser le point de congélation des hydrocarbures et trouve de nombreuses autres applications. Ainsi, la production annuelle de méthanol aux Etats-Unis est de 5 millions de $m^3$.

Généralement, le méthanol est produit industriellement par hydrogénation catalytique du monoxyde de carbone et différents catalyseurs ont été mis au point pour réaliser cette synthèse.

Ainsi, l'un des premiers catalyseurs fut l'oxyde de chrome et de zinc; l'un des plus récents est un mélange d'oxyde de cuivre et de zinc sur un support d'oxyde de chrome ou d'oxyde d'aluminium.

La réaction de synthése du méthanol:

$CO + 2H_2 \rightarrow CH_3OH$

qui paraît pourtant des plus simples, n'a pas encore été parfaitement élucidée au niveau du mécanisme réactionnel.

En effet, la variation $\Delta G$ de l'enthalpie libre est négative seulement à des températures inférieures à 140°C. Avec les catalyseurs usuels, une température bien supérieure de l'ordre de 200 à 300°C est nécessaire pour avoir une activité catalytique suffisante. Par ailleurs, une pression importante de l'ordre de 5 à 20 MPa est également nécessaire pour s'affranchir de la limitation thermodynamique du rendement.

Aussi, il serait d'un intérêt considérable de disposer de catalyseurs permettant de réaliser cette réaction à des températures et des pressions plus basses avec un rendement satisfaisant.

Jusqu'à présent, les recherches ont porté sur l'obtention de nouveaux catalyseurs métalliques tels que le platine, le palladium, l'iridium et le rhodium et de complexes métalliques tels que $Rh(CO)_5$ et $HCo(CO)_4$.

Cependant, de tels catalyseurs ne permettent pas de réaliser la synthèse du méthanol à la pression et à la température ambiantes.

La présente invention a précisément pour objet un procédé de préparation du méthanol au moyen d'un nouveau catalyseur organique qui permet de réaliser cette synthèse à la pression et à la température ambiantes.

Le procédé, selon l'invention, de préparation du méthanol par réaction du monoxyde de carbone avec de l'hydrogéne en présence d'un catalyseur, se caractérise en ce que l'on utilise comme catalyseur un polymère organique, conjugué conducteur de l'électricité.

Récemment, on a découvert que certains matériaux organiques étaient capables de conduire le courant électrique, et parmi ceux-ci on connaît des polymères organiques dopés tels que le polyacétylène, le polyphénylène et le polypyrrole. Jusqu'à présent, on a surtout utilisé des matériaux de ce type comme matières actives d'électrodes dans des générateurs électrochimiques. Mais selon l'invention, on a découvert que des matériaux de ce type constitués par des polymères organiques présentaient de plus des propriétés catalytiques, en présence ou en l'absence d'un champ électrique. Ainsi, selon l'invention, on utilise de tels polymères organiques comme catalyseur pour la synthèse du méthanol.

A titre d'exemples de polymères organiques susceptibles d'être utilisés dans l'invention, on peut citer le polyacétylène, le polyparaphénylène, le polysulfuru de phénylène, le polythiophène, la polyaniline, le polypyrrole et leurs dérivés substitués.

De préférence, delon l'invention, on utilise du polypyrrole répondant à la formule:

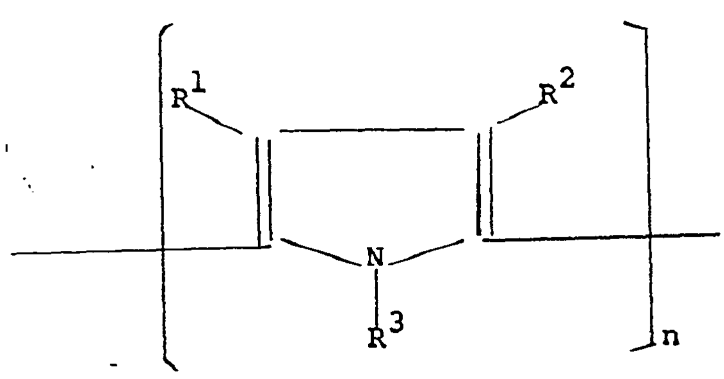

dans laquelle $R^1$ $R^2$ et $R^3$ qui peuvent être identiques ou différents, représentent un atome d'hydrogène, un groupement choisi parmi $NO_2$, $SO_2$, CN, $OCH_3$,

Cl, F, $C(=O)OH$, $C(=O)OR$, SCN, OCN, $C(=O)R$, $SO_2R$, $S(=O)R$, $P(=O)(OR)_2$,

SR avec R représentant un radical alkyle ou aryle, un groupement incluant un complexe d'un métal de transition ou un radical choisi parmi les radicaux alkyle et aryle comportant éventuellement un ou plusieurs substituants choisis dans le groupe comprenant $NO_2$,

$SO_2$, CN, $OCH_3$, Cl, F, SCN, OCN, $C(=O)OR$, $SO_2R$, $S(=O^-)OR$,
$C(=O)OH$, $C(=O)OR$, $P(=O)(OR)_2$,

SR avec R représentant un radical alkyle ou aryle, et un groupement incluant un radicomplexe d'un métal de transition, et n est un nombre superieur à 5.

De préférence n est compris entre 5 et 200 000.

Parmi les complexes de métaux de transition susceptibles d'être utilisés, on peut citer les complexes de ruthénium de formule:

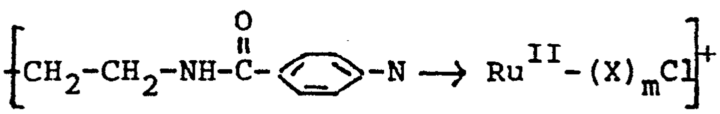

avec X représentant un dérivé tel qu'un dérivé azoté, notamment la pyridine ou la bipyridine et m étant compris entre 2 et 4.

Selon un premier mode de réalisation du procédé de l'invention, on effectue la réaction dans une cellule électrochimique contenant un électrolyte liquide et comportant une premiàre électrode comprenant le polymère organique conducteur qui constitue le catalyseur, en dispersant dans l'électrolyte, sous la forme d'un mélange gazeux, l'hydrogène et le monoxyde de carbone participant à la réaction, et en établissant une différence de potentiel entre ladite première électrode et une seconde électrode immergées dans l'électrolyte.

Dans ces conditions, il se produit des réactions électrochimiques au niveau des électrodes et ceci se traduit par un phénomène oscillatoire au niveau du courant qui traverse le circuit. On suppose que ces réactions sont les suivantes: il se produit tout d'abord une oxydation de l'un des gaz en phase adsorbée sur le polymère organique; le produit d'oxydation ainsi formé peut alors subir l'attaque nucléophile de l'autre gaz également en phase adsorbée sur le polymère organique, et il se produit ensuite une autre étape d'oxydation, puis une addition nucléophile et enfin une réduction électrochimique.

Ainsi, ces rèactions pourraient correspondre au mécanisme suivant:

$$CO \xrightarrow{-e} CO^{\cdot +}$$

$$CO^{\cdot +} + H_2 \longrightarrow COH_2^{\cdot +}$$

$$COH_2^{\cdot +} \longrightarrow COH^{\cdot} + H^+$$

$$COH^{\cdot} \xrightarrow{-e} COH^+$$

$$COH^+ + H_2 \longrightarrow CH_3O^+$$

$$CH_3O^+ \xrightarrow{+e} CH_3O^{\cdot}$$

$$CH_3O^{\cdot} + H^+ \longrightarrow CH_3OH^{\cdot +}$$

$$CH_3OH^{\cdot +} \xrightarrow{+e} CH_3OH$$

Les réactions ci-dessus indiquent que le bilan électrique est nul. Cependant, lorsqu'on applique une différence de potentiel entre les électrodes, qui conduit suivant le sens du courant à une oxydation ou plus particulièrement à une réduction, on améliore la vitesse de réaction et le rendement. En outre, en modifiant les débits relatifs de l'hydrogéne ou de l'oxyde de carbone, on peut encore améliorer le rendement.

Néanmoins, on peut aussi obtenir du méthanol sans passage de courant électrique en utilisant un polymère organique qui a été préalablement oxydé. Aussi, selon un second mode de réalisation du procédé de l'invention, on effectue la réaction dans une cellule contenant un électrolyte liquide dans lequel est immergé ledit polymère oxydé, en dispersant dans l'électrolyte liquide, sous la forme d'un mélange gazeux, l'hydrogène et le monoxyde de carbone participant à la réaction. Dans ces conditions, on obtient également du méthanol, mais le rendement de la réaction est inférieur à celui que l'on obtient avec passage d'un courant électrique dans la cellule.

Dans ces deux modes de réalisation du procédé de l'invention, l'électrolyte liquide est avantageusement une solution d'au moins un sel de lithium dans un solvant organique.

Les sels de lithium susceptibles d'être utilisés sont le perchlorate de lithium, le tétrafluoborate de lithium, le tétrachloro aluminate de lithium, l'hexafluorophosphate de lithium et l'hexafluoroarséniate de lithium.

On peut toutefois utiliser d'autres sels que les sels de lithium, par exemple des sels d'ammonium tels que le borofluorure de tétraéthylammonium et le chlorure de tétraéthylammonium.

Les solvants organiques utilisés peuvent également être très divers. A titre d'exemples de tels solvants, on peut citer les éthers linéaires tels que le diméthoxyéthane, les éthers cycliques tels que le tétrahydrofurane et le dioxolane, des esters comme le carbonate de propylane et l'acétonitrile. De préference, on utilise l'acétonitrile.

Par ailleurs, il est préférable d'utiliser un électrolyte acide car la réaction s'effectue beaucoup mieux en milieu acide. Aussi, l'électrolyte contient généralement $10^{-4}$ à $10^{-1}$ mol.1 $^{-1}$ d'un acide inorganique, par exemple d'acide perchlorique. En effet, si l'on réalise au début la réaction en milieu neutre non tamponné, il faut un temps d'induction de plusieurs heures d'oxydation avant d'obtenir du méthanol. Ceci peut s'expliquer par le fait qu'il faut former suffisamment de protons, grâce en particulier à l'oxydation de l'hydrogène pour que la réaction se fasse à une vitesse notable. On peut dans certains cas produire directement l'hydrogène à partir des protons obtenus par réduction de la solution.

Lorsque, selon le premier mode de réalisation du procédé de l'invention, on réalise la réaction dans une cellule électrochimique en faisant passer du courant à l'intérieur de la cellule, la cellule comprend, de préférence, également une électrode de référence à l'argent et la tension appliquée sur l'électrode comportant le polymère organique conducteur est, de préférence, de -0,1 à +0,5 V par rapport à cette électrode d'argent. La densité de courant est généralement de 1nA/cm$^2$ à 100mA/cm$^2$.

Dans le cas où l'on opère dans une cellule électrochimique, la premiére électrode qui comporte le polymère organique peut être constituée par un support en matériau conducteur de l'électricité ayant un potentiel d'oxydation supérieur à 0,7 V par rapport à l'électrode d'argent, ce support etant revêtu dudit polymère organique. Les supports susceptibles d'être utilisés sont le titane, l'acier inoxydable, le carbone, l'oxyde d'étain, l'oxyde d'indium et le platine. Ce support peut être revêtu d'une couche de polymère organique par électropolymérisation en déposant le polymère sur le support à partir d'une solution électrolytique contenant le monomère ou encore un oligomère, un électrolyte support et un solvant, en faisant passer un courant électrique entre le support et une contre-électrode immergée également dans la solution électrolytique. Pour cette électropolymérisation, on peut utiliser les électrolytes et les solvants mentionnés plus haut. Ce mode de préparation de l'électrode présente en particulier l'avantage de conduire directement à un polymàre organique dopé par l'anion du sel servant d'électrolyte support.

Lorsqu'on opère par voie électrochimique, on peut aussi utiliser une cellule électrochimique séparée en deux compartiments par une membrane semiperméable. Dans ce cas, le polymère organique conducteur est sous la forme de particules dispersées dans l'électrolyte de l'un desdits compartiments, et on introduit le mélange d'hydrogène et de monoxyde de carbone dans le compartiment où sont dispersées les particules de polymère organique.

Dans ce cas, le polymère organique peut être préparé, soit par voie électrochimique comme précédemment, soit par voie chimique, en utilisant des agents oxydants pour polymériser le monomère correspondant en solution dans un solvant approprié. Dans ce dernier cas, le polymère obtenu est précipité sous la forme d'une poudre. Les agents oxydants utilisés sont des agents dont le potentiel Rédox est proche de celui du monomère. Lorsque le monomère est le pyrrole, les agents oxydants peuvent être constitués par du perchlorate ferrique, du sulfate ferrique, du nitrate double d'ammonium et de cérium, du persulfate d'ammonium et des sels de cations ou radicaux cations organiques comme le perchlorate de méthyle-10-phénothiazinium.

Les solvants utilisés peuvent être tres divers. Ainsi, on peut utiliser de l'eau, des solutions aqueuses d'acide tel que l'acide sulfurique, l'acide perchlorique et des solvants organiques comme l'acétonitrile, le méthanol et le dichlorométhane.

Dans le second mode de mise en oeuvre du procédé de l'invention, c'est-à-dire lorsqu'on opère dans une cellule en l'absence de champ électrique, le polymère organique oxydé peut être préparé par électropolymérisation dans les conditions décrites précédemment. Ceci permet d'obtenir directement un polymère organique oxydé qui est dopé par l'anion du sel ayant servi d'électrolyte support pour la réaction d'électropolymérisation.

Dans ce cas, le polymère est déposé sur un support qui peut être constitué d'un matériau conducteur de l'électricité ayant un potentiel d'oxydation supérieur à 0,7 V par rapport à l'électrode d'argent, par exemple en acier inoxydable, en carbone, en oxyde d'étain en oxyde d'indium ou en platine. Dans ce second mode de réalisation, on peut aussi utiliser des particules de polymère obtenues par voie chimique et les soumettre à un traitement d'oxydation préalable par passage de courant dans une cellule électrolytique comportant un électrolyte contenant les particules de polymère maintenues à l'état de lit fluidisé lors du passage du courant. Le polymère peut être sous la forme de particules du seul polymère ou sous forme d'un support revêtu de polymère.

Selon un troisième mode de mise en oeuvre du procédé de l'invention, on réalise la réaction par voie sèche en l'absence de courant électrique. Dans ce cas, on effectue la réaction en mettant en contact un mélange gazeux de monoxyde de carbone et d'hydrogène avec un lit de particules dudit polymère organique conducteur de l'électricité qui a été préalablement oxydé.

Dans les trois modes de mise en oeuvre du procédé de l'invention, on peut améliorer la vitesse et le rendement en méthanol en réalisant la réaction à une température supérieure à la température ambiante, mais inférieure à la température de décomposition du polymère organique, par exemple à une température allant de 20 à 200°C. On peut aussi améliorer le rendement en opérant sous pression supérieure à la pression atmosphérique. Toutefois, pour des raisons économiques, on opère généralement à la pression atmosphérique.

D'autres caractéristiques et avantages de l'invention apparaîtront mieux à la lecture de la description qui suit donnée bien entendu à titre illustratif et non limitatif en référence au dessin annexé sur lequel:

- la figure 1 représente en coupe verticale une cellule electrochimique pour la mise en oeuvre du procédé de l'invention, et
- la figure 2 est une vue en perspective d'une autre cellule électrochimique susceptible d'être utilisée pour mettre en oeuvre le procédé de l'invention.

Sur la figure 1, on voit que la cellule électrochimique comprend un compartiment central 1 rempli d'électrolyte liquide qui est en communication par sa base avec un second compartiment 3 contenant une électrode de platine 5 constituant la deuxième électrode du dispositif et avec un troisième compartiment 7 contenant une électrode de référence à l'argent 9. Dans le compartiment central on peut introduire l'électrode 11 qui est une électrode en platine recouverte d'une couche d'un polymère organique conducteur et qui constitue ainsi la première électrode de la cellule électrochimique. Cette électrode est montée dans un support 13 comprenant une conduite 15 pour l'introduction des deux gaz participant à la réaction et cette conduite 15 débouche dans le compartiment central 1 juste en-dessous de l'électrode de platine 11 recouverte de polymère organique. Les gaz sont ainsi dispersés dans l'électrolyte du compartiment central et ils peuvent être évacués par le tube de sortie 17. L'exemple donné ci-après illustre l'utilisation d'une telle cellule électrochimique pour la préparation du méthanol.

**EXEMPLE 1**

Dans cet exemple, l'électrode 11 est une électrode Tacussel au platine non platiné qui est recouverte d'une couche de polypyrrole de 0,1 µm d'épaisseur et les compartiments 1, 3 et 7 sont remplis de 10 $cm^3$ d'acétonitrile 0,1 M en $LiClO_4$ et $10_{-2}$ M en $HClO_4$. L'électrode 9 est constituée par un fil d'argent et l'électrode 3 par un fil de platine.

L'électrode 11 a été recouverte de polypyrrole de formule:

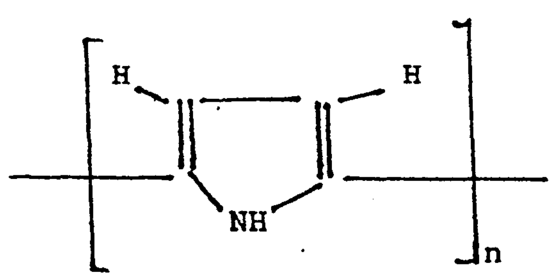

par électropolymérisation pendant trois minutes, avec une densité de courant de 0,2 $mA/cm^2$, d'une solution $6.10^{-3}$ M de pyrrole dans l'acétonitrile 0,1 M en $LiClO_4$

Pour réaliser la synthèse du méthanol, on introduit par la conduite 15 de l'hydrogène très pur (qualité N55 de l'Air Liquide qui correspond à un degré de pureté supérieur à 99,9995%) et du monoxyde de carbone très pur, (qualité N45 de l'Air Liquide ayant un degré de pureté supérieur à 99,9958). On détermine le débit des gaz par passage à travers un bulleur à l'huile silicone et on établit un débit d'hydrogène environ deux fois plus

important que le débit de monoxyde de carbone. Avant son entrée dans la conduite 15, le mélange gazeux a traversé un saturateur d'acétonitrile dans lequel le débit est d'environ une bulle toutes les cinq secondes.

On applique alors sur l'électrode 11 qui constitue l'anode une tension de 0,3 V par rapport à l'électrode 9 à l'argent pendant 4 heures. On observe sur le microampèremètre de contrôle un courant oscillant qui traverse le circuit. Le bilan coulométrique est extrêmement faible et il est positif de près de 0,2 coulomb. En fin d'opération, on analyse l'acétonitrile présent dans la cellule par chromatographie en phase gazeuse sur une colonne PORAPAK Q. On détecte à la sortie de la colonne du méthanol qui passe juste avant l'acétonitrile. La quantité de méthanol produite correspond à une quantité voisine de 5 µl pour les 10 $cm^3$ d'acétonitrile en opérant à 20° C sous la pression atmosphérique. Si l'on porte la température à 40° C et la pression à 0,2 MPa, on obtient une quantité de méthanol voisine de 25 µl.

Ainsi, le procédé de l'invention permet de réaliser la synthèse du méthanol à pression et température ambiantes.

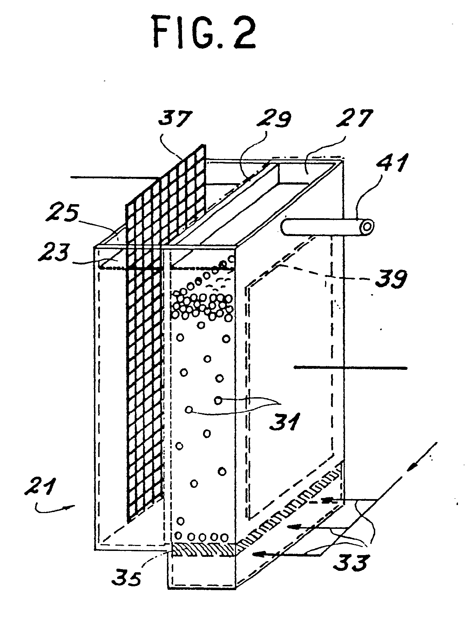

Sur la figure 2, on a représenté un autre mode de réalisation de la cellule électrochimique susceptible d'être utilisée pour la synthèse du méthanol.

Dans ce cas, la cellule comprend une cuve 21 remplie d'électrolyte 23 et séparée en deux compartiments 25 et 27 par une membrane semi-perméable 29 réalisée par exemple en Nafion $^R$. Dans le compartiment 27 sont dispersées des particules 31 de polymère organique qui sont maintenues dans l'électrolyte à l'état de lit fluidisé grâce à l'introduction d'un gaz par la conduite 33 débouchant à la base du compartiment en-dessous d'une grille 35 destinée à retenir les particules 31 de polymère organique lorsqu'elles ne sont pas maintenues à l'état de lit fluidisé. La cellule comprend de plus une électrode 37 immergée dans le compartiment 25 et une électrode 39 disposée sur la paroi du compartiment 27. Les gaz introduits par la conduite 33 sont évacués par la conduite 41.

L'exemple suivant illustre le fonctionnement de cette cellule électrochimique.

**EXEMPLE 2**

Dans cet exemple, les particules 31 sont constituées par des particules de polypyrrole de formule:

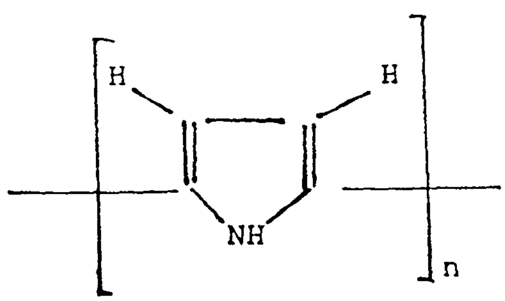

qui ont été préparées par voie chimique en réalisant l'oxydation du pyrrole en solution dans de l'acétonitrile au moyen de perchlorate ferrique. L'électrolyte 23 est constitué par de l'acétonitrile 0,01 M en $HClO_4$ et 0,1 M en $LiClO_4$. La quantité de particules de polypyrrole utilisée est de 1,3 g et la quantité d'électrolyte de 170 $cm^3$. On établit entre les électrodes 37 et 39 une différence de potentiel de 0,2 V pendant 5 heures. Comme précédemment, on analyse en fin d'opération l'électrolyte par chromatographie en phase gazeuse pour déterminer sa teneur en méthanol. On obtient, dans ces conditions, une quantité de méthanol voisine de 80 µl lorsque l'on opère à la pression et à la température ambiantes.

Si l'on opère à une températrue de 40° C sous 0,2 MPa, la quantité de méthanol est proche de 0,5 $cm^3$.

**EXEMPLE 3**

Cet exemple illustre la préparation de méthanol en l'absence de champ électrique. Dans ce cas, on utilise les dispositifs de la figure 2, mais sans établir de différence de potentiel entre les électrodes 37 et 39. Le polypyrrole a été préalablement oxydé par passage de courant avant d'introduire le mélange d'hydrogène et de monoxyde de carbone. Dans ces conditions, au bout de 5 heures de traitement on obtient 60 µl de méthanol à 20° C, sous 0,1 MPa.

On remarque ainsi que le rendement est moins important lorsqu'on opère en l'absence d'un champ électrique.

**EXEMPLE 4**

Cet exemple illustre la préparation de méthanol en phase gazeuse selon le troisième mode de mise en oeuvre du procédé de l'invention.

Dans ce cas, on opère sous une pression de 0,5 MPa à une température de 200° C et on fait passer le mélange de monoxyde de carbone et d'hydrogène sur un lit de 1 g de particules de polypyrrole préparé par voie chimique comme dans l'exemple 2 qui a été préalablement oxydé comme dans l'exemple 3. A la sortie, on condense le gaz par passage dans un réfrigérant à l'azote liquide et on recueille ainsi 0,1 $cm^3$ de méthanol après 5 heures de réaction.

## Revendications

1. Procédé de préparation du méthanol par réaction du monoxyde de carbone avec de l'hydrogène en présence d'un catalyseur, caractérisé en ce que l'on utilise comme catalyseur un polymère organique conducteur de l'électricité.

2. Procédé selon la revendication 1, caractérisé en ce que le polymère organique est choisi dans le groupe comprenant le polyacétylène, le polyparaphénylène, le polysulfure de phénylène, le polythiophène, la polyaniline, le polypyrrole et leurs dérivés substitués.

3. Procédé selon la revendication 1, caractérisé en ce que le catalyseur est du polypyrrole répondant à la formule:

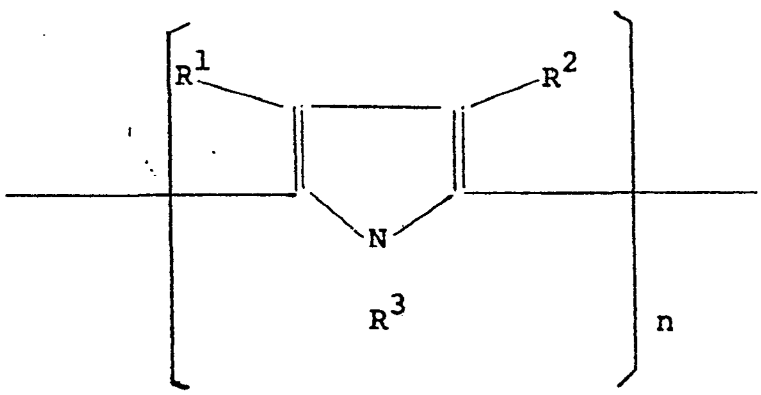

dans laquelle $R^1$, $R^2$ et $R^3$ qui peuvent être identiques ou différents, représentent un atome d'hydrogène, un groupement choisi parmi $NO_2$, $SO_2$, CN, $OCH_3$,

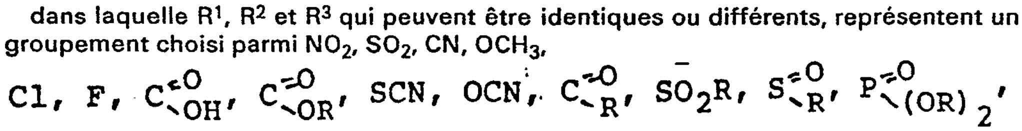

SR avec R représentant un radical alkyle ou aryl, un groupement incluant un complexe d'un métal de transition, ou un radical choisi parmi les radicaux alkyle et aryle comportant éventuellement un ou plusieurs substituants choisis dans le groupe comprenant $NO_2$,

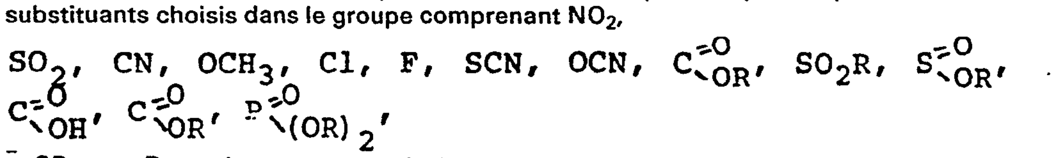

SR avec R représentant un radical alkyle ou aryle, et un groupement incluant un complexe d'un métal de transition et n est un nombre supérieur à 5.

4. Procédé selon la revendication 3, caractérisé en ce que n est compris entre 5 et 200 000.

5. Procédé selon l'une quelconque des revendications 3 et 4, caractérisé en ce que le complexe d'un métal de transition est un complexe de ruthénium de formule:

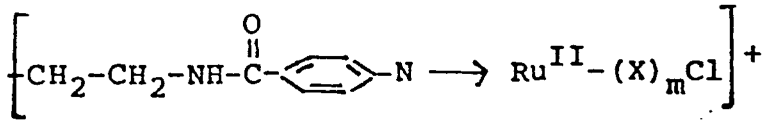

avec X représentant un dérivé azoté, notamment la pyridine ou la bipyridine, et m étant compris entre 2 et 4.

6. Procédé selon l'une quelconque des revendications 1 à 5, caractérisé en ce que l'on effectue la réaction dans une cellule électrochimique contenant un électrolyte liquide et comportant une première électrode (11, 39) comprenant le polymère organique conducteur qui constitue le catalyseur, en dispersant dans l'électrolyte, sous la forme d'un mélange gazeux, l'hydrogène et le monoxyde de carbone participant à la réaction, et en établissant une différence de potentiel entre ladite première électrode (11, 39) et une seconde électrode (5, 37) immergées dans l'électrolyte.

7. Procédé selon l'une quelconque des revendications 1 à 5, caractérisé en ce que ledit polymère organique est sous forme oxydée et en ce que l'on effectue la réaction dans une cellule contenant un électrolyte liquide dans lequel est immergé ledit polymère oxydé, en dispersant dans le milieu liquide, sous la forme d'un mélange gazeux, l'hydrogène et le monoxyde de carbone participant à la réaction.

8. Procédé selon l'une quelconque des revendications 6 et 7, caractérisé en ce que l'électrolyte liquide est une solution d'au moins un sel de lithium dans un solvant organique.

9. Procédé selon la revendication 8, caractérisé en ce que le sel de lithium est le perchlorate de lithium, le tétrafluoborate de lithium, le tétrachloroaluminate de lithium, l'hexafluorophosphate de lithium ou l'hexafluoroarséniate de lithium.

10. Procédé selon la revendication 8, caractérisé en ce que le solvant organique est l'acétonitrile.

11. Procédé selon l'une quelconque des revendications 6 à 10, caractérisé en ce que l'électrolyte contient $10^{-4}$ à $10^{-1}$ $mol.l^{-1}$ un acide inorganique.

12. Procédé selon la revendication 11, caractérisé en ce que l'acide inorganique est l'acide perchlorique.

13. Procédé selon la revendication 6, caractérisé en ce que la cellule comprend de plus une électrode de référence à l'argent et en ce que la tension appliquée sur l'électrode comprenant le polymère organique conducteur est de -0,1 V à +0,5 V par rapport à cette électrode d'argent.

14. Procédé selon la revendication 6, caractérisé en ce que la densité de courant est de $1nA/cm^2$ à 100 $mA/cm^2$.

15. Procédé selon la revendication 6, caractérisé en ce que la cellule électrochimique est séparée en deux compartiments (25, 27) par une membrane semi-perméable (29), en ce que le polymère organique est sous la forme de particules (31) dispersées dans l'électrolyte de l'un desdits compartiments et en ce que l'on introduit le mélange d'hydrogène et de monoxyde de carbone dans le compartiment où sont dispersées les particules de polymère organique.

16. Procédé selon la revendication 6, caractérisé en ce que la première électrode (11) est constituée par un support en matériau conducteur de l'électricité ayant un potentiel d'oxydation supérieur à 0,7 V par rapport à une électrode d'argent, ledit support étant revêtu dudit polymère organique.

17. Procédé selon l'une quelconque des revendications 1 à 4, caractérisé en ce que l'on effectue la réaction en mettant en contact un mélange gazeux de monoxyde de carbone et d'hydrogène avec un lit de particules dudit polymère organique conducteur de l'électricité qui a été préalablement oxydé.

18. Procédé selon l'une quelconque des revendications 1 à 17, caractérisé en ce que l'on opère à une température allant de la température ambiante à 200°C.

19. Procédé selon l'une quelconque des revendications 1 à 18, caractérisé en ce que l'on effectue la réaction sous une pression supérieure à la pression atmosphérique.

20. Procédé selon l'une quelconque des revendications 6 à 19, caractérisé en ce que le polymère organique répond à la formule:

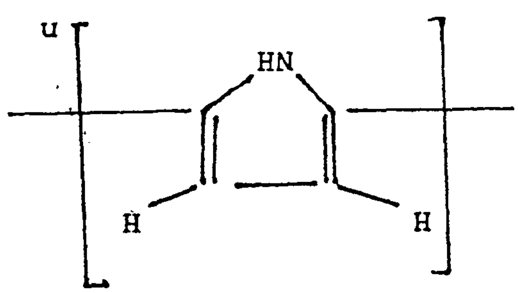

avec n compris entre 5 et 200 000.

**Patentansprüche**

1. Verfahren zur Herstellung von Methanol durch Umsetzung von Kohlenmonoxid mit Wasserstoff in Gegenwart eines Katalysators, dadurch gekennzeichnet, daß man als Katalysator ein elektrisch leitendes organisches Polymeres verwendet.

2. Verfahren nach Anspruch 1, dadurch gekennzeichnet, daß das organische Polymere ausgewählt wird aus der Gruppe, die umfaßt Polyacetylen, Polyparaphenylen, Phenylenpolysulfid, Polythiophen, Polyanilin, POlypyrrol und ihre substituierten Derivate.

3. Verfahren nach Anspruch 1, dadurch gekennzeichnet, daß es sich bei dem Katalysator um Polypyrrol der Formel handelt

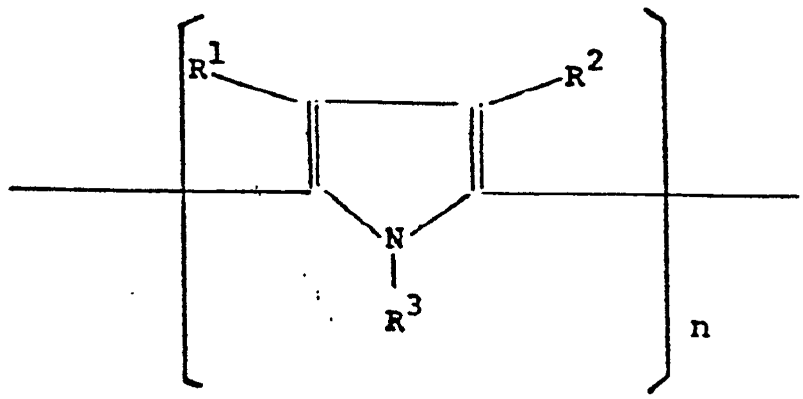

worin $R^1$, $R^2$ und $R^3$, die gleich oder verschieden sein können, darstellen ein Wasserstoffatom, eine Gruppe, die ausgewählt wird aus $NO_2$, $SO_2$, CN, $OCH_3$, Cl, F, $C<^{O}_{OH}$, $C<^{O}_{OR}$, SCN, OCN, $C<^{O}_{R}$, $SO_2R$, $S<^{O_2}_{R}$, $P<^{O}_{(OR)_2}$, SR,

worin R darstellt einen Alkyl- oder Arylrest, eine Gruppe die umfaßt einen Komplex eines Übergangsmetalls, oder einen Rest, der ausgewählt wird aus Alkyl- und Arylresten, die ggf. einen oder mehrere Substituenten tragen, die ausgewählt werden aus der Gruppe, die umfaßt $NO_2$, $SO_2$, CN, $OCH_3$, Cl, F, SCN, OCN, $C<^{O}_{OR}$, $SO_2R$, $SO_2R$, $S<^{O}_{OR}$, $C<^{O}_{OH}$, $C<^{O}_{OR}$, $P<^{O}_{(OR)_2}$, SR,

wobei R darstellt einen Alkyl- oder Arylrest, und eine Gruppe, die einen Komplex eines Überganqsmetalls umfaßt, und worin n eine Zahl größer als 5 bedeutet.

4. Verfahren nach Anspruch 3, dadurch gekennzeichnet, daß n zwischen 5 und 200 000 liegt.

5. Verfahren nach einem der Ansprüche 3 und 4, dadurch gekennzeichnet, daß es sich bei dem Komplex eines Übergangsmetalls um einen Rutheniumkomplex der Formel handelt

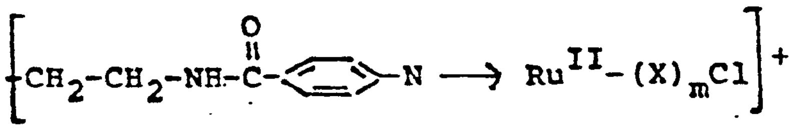

worin K ein stickstoffhaltiges Derivat, insbesondere Pyridin oder Bipyridin, und m eine Zahl zwischen 2 und 4 bedeuten.

6. Verfahren nach einem der Ansprüche 1 bis 5, dadurch gekennzeichnet, daß man die Reaktion in einer elektrochemischen Zelle durchführt, die einen flüssigen Elektrolyten enthält und eine erste Elektrode (11, 39) aufweist, die das elektrisch leitende organische Polymere enthält, das den Katalysator darstellt, indem man in dem Elektrolyten in Form einer Gasmischung Wasserstoff und Kohlenmonoxid, die an der Reaktion teilnehmen, dispergiert und eine Potentialdifferenz zwischen der ersten Elektrode (11, 39) und einer zweiten, in den Elektrolyten eingetauchten Elektrode (5, 37) einstellt.

7. Verfahren nach einem der Ansprüche 1 bis 5, dadurch gekennzeichnet, daß das organische Polymere in oxidierter Form vorliegt und daß man die Reaktion in einer Zelle durchführt, die einen flüssigen Elektrolyten enthält, in den das oxidierte Polymere eingetaucht ist, indem man in dem flüssigen Milieu in Form einer Gasmischung den Wasserstoff und das Kohlenmonoxid, die an der Reaktion teilnehmen, dispergiert.

8. Verfahren nach einem der Ansprüche 6 und 7, dadurch gekennzeichnet, daß es sich bei dem flüssigen Elektrolyten um eine Lösung mindestens eines Lithiumsalzes in einem organischen Lösungsmittel handelt.

9. Verfahren nach Anspruch 8, dadurch gekennzeichnet, daß es sich bei dem Lithiumsalz um Lithiumperchlorat, Lithiumtetrafluoborat, Lithiumtetrachloroaluminat, Lithiumhexafluorophosphat oder Lithiumhexafluoroarsenat handelt.

10. Verfahren nach Anspruch 8, dadurch gekennzeichnet, daß es sich bei dem organischen Lösungsmittel um Acetonitril handelt.

11. Verfahren nach einem der Ansprüche 6 bis 10, dadurch gekennzeichnet, daß der Elektrolyt $10^{-4}$ bis $10^{-1}$ Mol/l einer anorganischen Säure enthält.

12. Verfahren nach Anspruch 11, dadurch gekennzeichnet, daß es sich bei der anorganischen Säure um Perchlorsäure handelt.

13. Verfahren nach Anspruch 6, dadurch gekennzeichnet, daß die Zelle außerdem eine Silberbezugselektrode aufweist und daß die an die das elektrisch leitende organische Polymere enthaltende Elektrode angelegte Spannung -0,1 V bis +0,5 V, bezogen auf die Silberelektrode, beträgt.

14. Verfahren nach Anspruch 6, dadurch gekennzeichnet, daß die Stromdichtel nA/cm$^2$ bis 100mA/cm$^2$ beträgt.

15. Verfahren nach Anspruch 6, dadurch gekennzeichnet daß die elektrochemische Zelle durch eine semipermeable Membran (29) in zwei Abteile (25, 27) unterteilt ist, daß das organische Polymere in Form von Teilchen (31) in dem Elektrolyten eines Abteils dispergiert ist und daß man die Mischung aus Wasserstoff und Kohlenmonoxid in das Abteil einleitet, in dem die organischen Polymerteilchen dispergiert sind.

16. Verfahren nach Anspruch 6, dadurch gekennzeichnet, daß die erste Elektrode (11) besteht aus einem Träger aus einem elektrisch leitenden Material mit einem Oxidationspotential von über 0,7 V, bezogen auf eine Silberelektrode, der von dem organischen Polymeren überzogen ist.

17. Verfahren nach einem der Ansprüche 1 bis 4, dadurch gekennzeichnet, daß man die Reaktion durchführt, indem man eine Gasmischung aus Kohlenmonoxid und Wasserstoff mit einem Teilchenbett des elektrisch leitenden oiganischen Polymeren, das vorher oxidiert worden ist, in Kontakt bringt.

18. Verfahren nach einem der Ansprüche 1 bis 17, dadurch gekennzeichnet, daß man bei einer Temperatur arbeitet, die von Umgebungstemperatur bis 200° C geht.

19. Verfahren nach einem der Ansprüche 1 bis 18, dadurch gekennzeichnet, daß man die Reaktion unter einem Druck oberhalb Atmosphärendruck durchführt.

20. Verfahren nach einem der Ansprüche 6 bis 19, dadurch gekennzeichnet, daß das organische Polymere die Formel hat

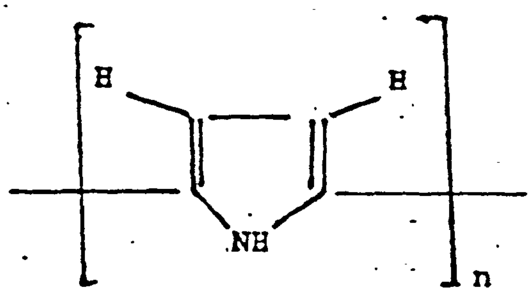

worin n eine Zahl zwischen 5 und 200 000 ist.

## Claims

1. Process for the preparation of methanol by reacting carbon monoxide with hydrogen in the presence of a catalyst, characterized in that an organic polymer which is an electrical conductor is used as the catalyst.

2. Process according to Claim 1, characterized in that the organic polymer is chosen from the group consisting of polyacetylene, polyparaphenylene, polyphenylenesulphide, polythiophene, polyaniline, polypyrrole and their substituted derivatives.

3. Process according to Claim 1, characterized in that the catalyst is polypyrrole corresponding to the formula:

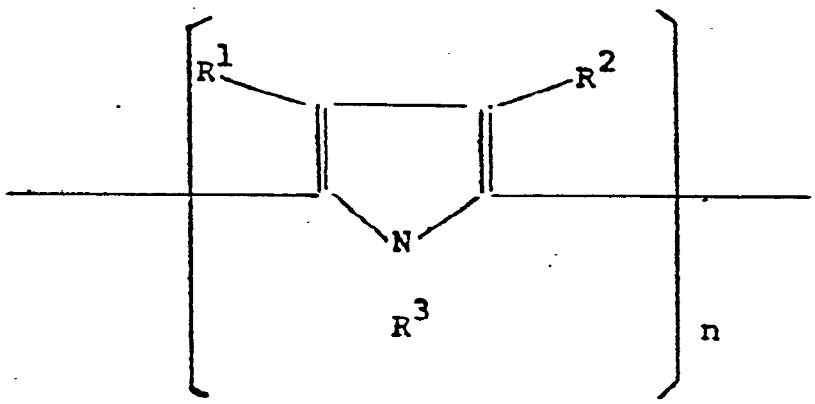

in which $R^1$, $R^2$ and $R^3$, which may be identical or different, represent a hydrogen atom, a group chosen from $NO_2$,

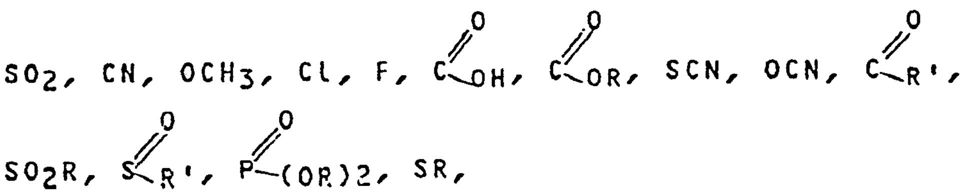

where R represents an alkyl or aryl radical, a group which includes a transition metal complex, or a radical chosen from the alkyl and aryl radicals, containing, if appropriate, one or more substituents chosen from the group which consists of $NO_2$, $SO_2$,

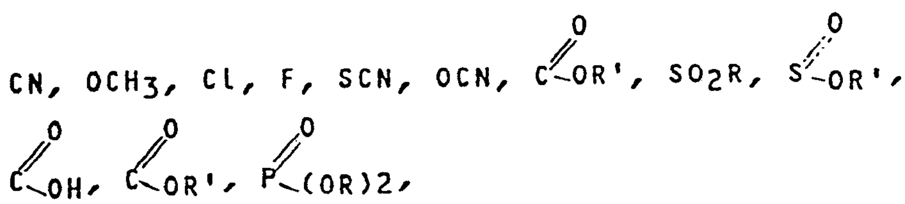

SR where R represents an alkyl or aryl radical, and a group which includes a transition metal complex and n is a number greater than 5.

4. Process according to Claim 3, characterized in that n is between 5 and 200,000.

5. Process according to either one of Claims 3 and 4, characterized in that the transition metal complex is a ruthenium complex of formula:

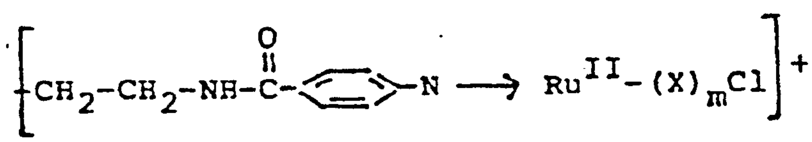

where X represents a nitrogenous derivative, especially. pyridine or bipyridine, and m is between 2 and 4.

6. Process according to any one of Claims 1 to 5, characterized in that the reaction is carried out in an electrochemical cell containing a liquid electrolyte and consisting of a first electrode (11, 39) containing the conducting organic polymer which forms the catalyst, by dispersing hydrogen and carbon monoxide, which take part in the reaction, in the form of a gaseous mixture, in the electrolyte, and by setting up a potential difference between the said first electrode (11, 39) and a second electrode (5, 37) which are immersed in the electrolyte.

7. Process according to any one of Claims 1 to 5, characterized in that the said organic polymer is in the oxidized form and in that the reaction is carried out in a cell containing a liquid electrolyte in which the said oxidized polymer is immersed, by dispersing hydrogen and carbon monoxide which take part in the reaction, in the form of a gaseous mixture, in the liquid medium.

8. Process according to either one of Claims 6 and 7, characterized in that the liquid electrolyte is a solution of at least one lithium salt in an organic solvent.

9. Process according to Claim 8, characterized in that the lithium salt is lithium perchlorate, lithium tetrafluoroborate, lithium tetrachloroaluminate, lithium hexafluorophosphate or lithium hexafluoroarsenate.

10. Process according to Claim 8, characterized in that the organic solvent is acetonitrile.

11. Process according to any one of Claims 6 to 10, characterized in that the electrolyte contains $10^{-4}$ to $10^{-1}$ mol.l$^{-1}$ of an inorganic acid.

12. Process according to Claim 11, characterized in that the inorganic acid is perchloric acid.

13. Process according to Claim 6, characterized in that the cell additionally contains a silver reference electrode and in that the voltage applied to the electrode containing the conducting organic polymer is from -0.1 V to +0.5 V relative to this silver electrode.

14. Process according to Claim 6, characterized in that the current density is 1nA/cm$^2$ to 100mA/cm$^2$.

15. Process according to Claim 6, characterized in that the electrochemical cell is separated into two compartments (25, 27) by a semi-permeable membrane (29), in that the organic polymer is in the form of particles (31) dispersed in the electrolyte of one of the said compartments and in that the mixture of hydrogen and carbon monoxide is introduced into the compartment in which the organic polymer particles are dispersed.

16. Process according to Claim 6, characterized in that the first electrode (11) consists of a support made of an electricity-conducting material which has an oxidation potential greater than 0.7 V relative to a silver electrode, the said support being coated with the said organic polymer.

17. Process according to any one of Claims 1 to 4, characterized in that the reaction is carried out by bringing a gaseous mixture of carbon monoxide and hydrogen into contact with a bed of particles of the said electricityconducting organic polymer which has previously been oxidized.

18. Process according to any one of Claims 1 to 17, characterized in that it is carried out at a temperature ranging from the ambient temperature to 200° C.

19. Process according to any one of Claims 1 to 18, characterized in that the reaction is carried out at a pressure greater than atmospheric pressure.

20. Process according to any one of Claims 6 to 19, characterized in that the organic polymer corresponds to the formula:

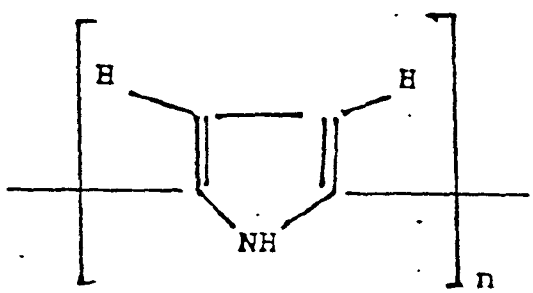

where n is between 5 and 200,000.

FIG.1

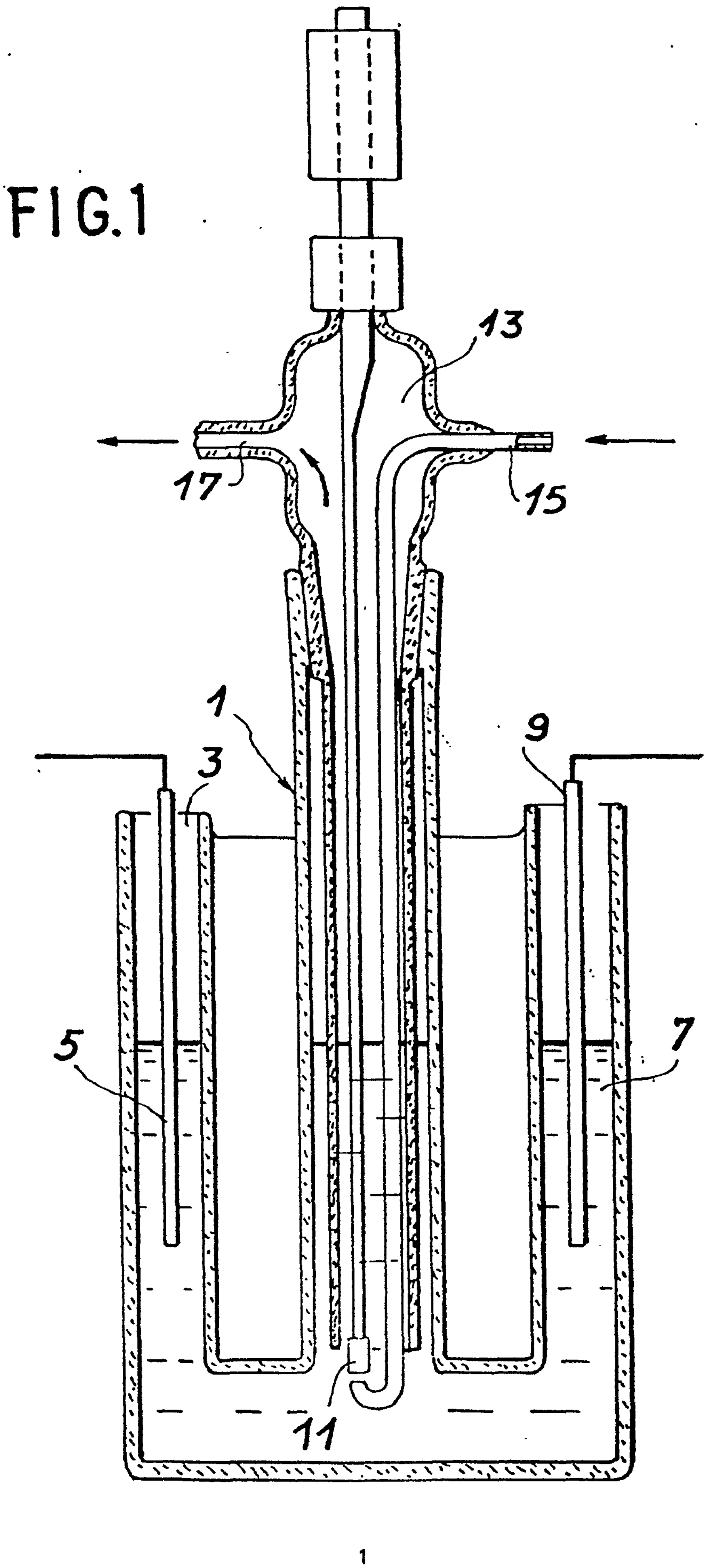

FIG. 2